# EUROPEAN PATENT SPECIFICATION

(11) **EP 0 711 782 B1**
(45) Date of publication and mention of the grant of the patent: **24.05.2000**
(21) Application number: 95115468.1
(22) Date of filing: 29.09.1995
(51) Int. Cl.: C07H 3/06

(54) **A method of preparing ganglioside**
Verfahren zur Herstellung von Gangliosiden
Procédé pour la préparation de gangliosides

(30) Priority: 30.09.1994 JP 26196694
(43) Date of publication of application: 15.05.1996
(73) Proprietor: SNOW BRAND MILK PRODUCTS CO., LTD., Sapporo-shi, Hokkaido 065 (JP)
(72) Inventor: Hanagata, Goro, Sayama-shi, Saitama 350-13 (JP); Miura, Susumu, Kawagoe-shi, Saitama 350 (JP); Tatsumi, Kiyoshi, Iruma-shi, Saitama 358 (JP)
(74) Representative: Splanemann Reitzner Baronetzky Westendorp Patentanwälte

(56) References cited:
- EP-A- 0 319 890
- AU-A- 3 200 493
- DE-A- 3 624 816
- J. BIOCHEM., vol. 98, 1985 pages 545-9, I. ISHIZUKA 'Acidic Glycolipids from Dolphin Kidney'
- J. CHROMATOGRAPHY, vol. 377, 1986 pages 59-67, A. LAEGREID ET AL. 'Purification of Human Milk Gangliosides by Silica Gel Chromatography'
- J. LIQUID CHROMATOGRAPHY, vol. 13, 1990 pages 1921-31, P. ILINOV ET AL. 'Direct Thin-Layer Chromatographic Method for Isolation of Gangliosides'

## Description

### BACKGROUND OF THE INVENTION

### FIELD OF THE INVENTION

The present invention relates to a method of preparing Ganglioside. In particular, the present invention relates to a method of preparing Ganglioside G_{M3} from Ganglioside G_{D3} included in a raw material containing lipids in situ.

The Ganglioside G_{M3} prepared by the method of the present invention is useful as a material for the manufacture of pharmaceuticals, cosmetics, foods, beverages and animal feeds.

### DESCRIPTION OF THE PRIOR ART

Ganglioside is a general name of sphingoglycolipid having sialic acid, and the existence of various molecular species thereof are known. Among them, Ganglioside G_{M3} has a structure in which reducing terminal of lactose bonds to a ceramide and a sialic acid α2-3 bonds to nonreducing terminal of the lactose. Ganglioside G_{D3} has a structure in which a further sialic acid α2-8 bonds to nonreducing terminal of Ganglioside G_{M3}, and thus the whole molecule of Ganglioside G_{D3} has two molecules of sialic acid.

As for Ganglioside G_{M3}, various physiological functions have been known, and for example proliferation inhibiting function of keratinocytes, angiogenic repressing function and differentiation function of leukocytes have been reported. In addition, Ganglioside G_{M3} has been known to be a receptor to influenza or Newcastle disease viruses.

As a method of preparing Ganglioside G_{M3} from Ganglioside G_{D3}, a method of hydrolyzing Ganglioside G_{D3} with sialidase or a method of hydrolyzing Ganglioside G_{D3} with an acid is known (Japanese Patent Application JP-A-5-279379; AU-B-661 089). However, the method of hydrolyzing with sialidase is not always considered to be a industrially advantageous method, since Ganglioside is inhibited by coexisting proteins, substrates are easily inhibited by coexisting free sialic acid and bonding sialic acid and the like, and the sialidase used is expensive. In addition, with the method of hydrolyzing with an acid, precipitates of proteins are easily produced although the cost needed for the conversion is low. It is, therefore, very difficult to apply this method to a raw material containing a high amount of proteins such as bovine milk or milk products. Further, there is the problem that viscosity of the solution is easily increased when hydrolyzed with an acid.

It is known that Ganglioside exists in milk fat globule membrane, animal cells or animal brains, and that among them Ganglioside G_{D3} exists in milk fat globule membrane in a high amount. The material containing a high amount of Ganglioside G_{D3} generally contains a high amount of lipids such as triglyceride or phospholipid since the milk fat globule membrane is contained in milk products, in particular, in cream or processed cream products, in a high amount.

EP-A-0 319 890 discloses the production of monosialoganglioside by autolysis, after homogenizing the raw material of beef brain in an acid buffer at a pH ranging from 4 to 5. There is no disclosure that G_{M3} can be effectively produced.

DE-A-3 624 816 discloses a method of producing monosialoganglioside wherein Ganglioside is heated in an aqueous medium at a temperature of more than 50°C and at a pH of 3.5 to 7. The starting materials for the Ganglioside are the brains of beef, pigs and the like.

### SUMMARY OF THE INVENTION

The present invention relates a method of preparing Ganglioside G_{M3} which comprises converting Ganglioside G_{D3} included in a raw material containing lipids into Ganglioside G_{M3} in situ at a pH ranging from 6 to 8, wherein the raw material containing lipids is cream or a milk product prepared from cream, and wherein the conversion is carried out by thermally treating the raw material.

By this method, only one sialic acid molecule bond to the nonreducing terminal of Gengloiside G_{D3} is desialized at a substantially neutral pH without the need of the addition of an acid or an alkali.

### BRIEF DESCRIPTION OF THE DRAWINGS

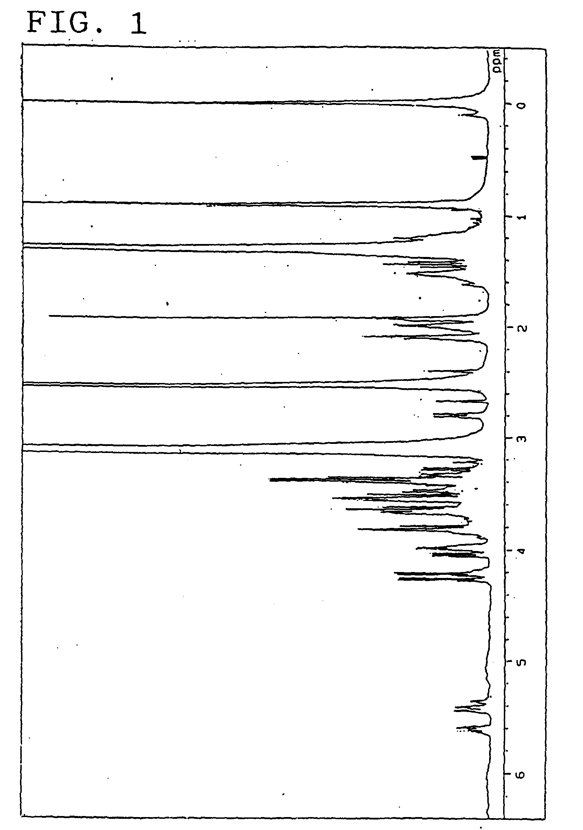

Fig.1 shows a NMR spectrum of the reaction product obtained in EXAMPLE 1.

Fig.2 shows the result of thin layer chromatography of the reaction product obtained in EXAMPLE 2.

### DETAILED DESCRIPTION OF THE PREFERRED EMBODIMENTS

As the raw material containing lipids used in the present invention, a raw material containing preferably 10 wt% or more lipids per solids is used, namely cream or milk products prepared from cream. In addition, a raw material containing lipids including Ganglioside G_{D3}, obtained from fresh milk, cream, butter milk, whey protein concentrates (WPC) or whey cream obtained by a method of extracting it from a raw material containing Ganglioside G_{D3} with an organic solvent such as ethanol (Japanese Patent Application JA-A-2-207090) may be used. The term lipids used herein means triglyceride, phospholipid, glycolipid or materials which may be extracted with a polar solvent such as chloroform-methanol mixture, and further means fat soluble materials such as carotenoid or sterol.

The thermal treatment is preferably carried out at a higher temperature than the temperatures usually adopted to heat foods or milk products. In the method of the present invention, by heating the raw material in a neutral pH zone ranging from 6 to 8, only one molecule of sialic acid can be desialized from Ganglioside G_{D3} to obtain Ganglioside G_{M3}. If the heating is continued, the produced Ganglioside G_{M3} will further be desialized to liberate the remained sialic acid. The higher the reaction temperature, the higher the production rate of Ganglioside G_{M3} becomes, but the higher the decomposition rate of Ganglioside G_{M3} becomes as well. Thus the reaction temperature is preferably set in the range from 60°C to 140°C, more preferably set in the range from 80°C to 110°C depending on the raw material used, and a suitable reaction time may be determined depending on the temperature in order to obtain Ganglioside G_{M3} in a high yield. The heat sterilization temperature usually adopted is within the above preferable range but heating time is longer than the above preferable time, so that the produced Ganglioside G_{M3} decomposes finally and thus this heating condition of the heat sterilization is not considered to be appropriate.

The Ganglioside prepared by the method of the present invention can be used as a material for the manufactures of pharmaceuticals, cosmetics, foods, beverages and animal feeds as it is, i.e. in a form included in the raw material containing lipids, or after separating or purifying it by a conventional method.

The following examples are further illustrative of the present invention, and are not to be construed as limiting the scope thereof. Unless expressly indicated to be otherwise, all parts and percentages are by weight.

### EXAMPLE 1

2L of 80% ethanol was added to 100g of butter milk powder, and the mixture was stirred at room temperature for 8 hours. Then precipitates were removed by filtration. Water was added, and the mixture was distilled under a reduced pressure at a low temperature repeatedly to remove ethanol to finally obtain 2L of an aqueous solution which was used as a starting material. The aqueous solution contained 2.6g/L of Ganglioside G_{D3} and 0.16g/L Ganglioside G_{M3}. The pH of the aqueous solution was 6.8.

The aqueous solution was heated to 95°C for 60 minutes to carry out the reaction. After completing the reaction, the reaction mixture was analyzed by thin layer chromatography (No.13749; obtained from Merck). The thin layer chromatogram was developed using a solvent (chloroform:methanol:H₂O=60:35:8, by vol.) and was color developed with resorcinol. It was confirmed that the reaction mixture contained 0.95g/L of Ganglioside G_{M3}.

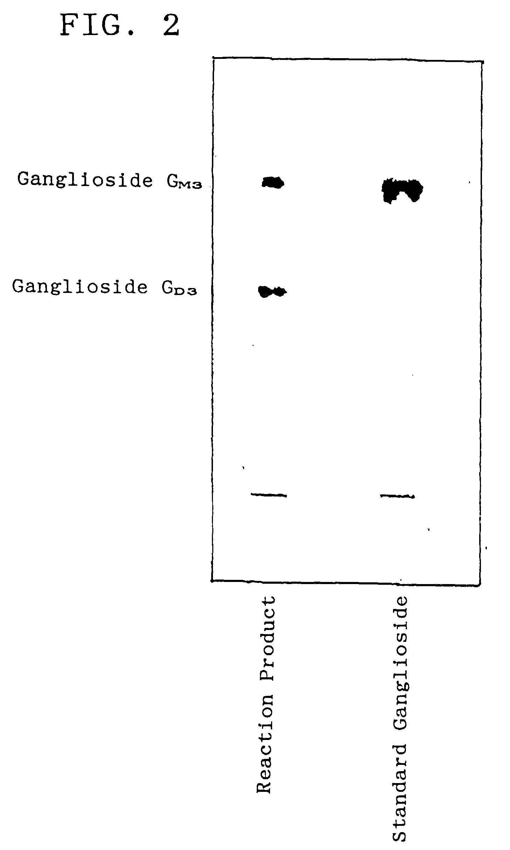

The reaction mixture was dried under a reduced pressure to obtain a white powder. The powder was analyzed by ion-exchange chromatography with DEAE-Sephadex A-25 (obtained from Pharmacia), which was followed by silica gel chromatography by Iatrobeads 6RS 8060 (obtained from Iatron Laboratories, Inc.) to collect a reaction product. After freeze drying the product, 0.90g of the obtained white powder was analyzed with NMR to obtain the result shown in Fig. 2. The result corresponded to the standard.

### EXAMPLE 2

2L of cream containing 48mg/L of Ganglioside _{D3} was heated to 85°C for 60 minutes to carry out the reaction. After completing the reaction, a lipid fraction was extracted from the reaction mixture with a solvent (chroloform:methanol=2:1), and was followed by thin layer chromatography as described in EXAMPLE 1. The result was as shown in Fig.2. It was proved that Ganglioside G_{M3} having the same Rf values as the Ganglioside G_{M3} derived from bovine brain. In addition, it was confirmed that the reaction mixture contained 15mg/L of Ganglioside G_{M3} by densitometry.

By the method of the present invention, Ganglioside G_{M3} can be prepared from Ganglioside G_{D3} inexpensively and simply without the need of pH setting with an acid or an alkali. Thus the method of the present invention is useful as an industrial method of preparing Ganglioside G_{M3}. In addition, since the Ganglioside G_{M3} prepared by the method of the present invention has physiological functions such as infection protective ability, it is useful as a raw material for the manufactures of pharmaceuticals, cosmetics, foods, beverages and animal feeds.

## Claims

1. A method of preparing Ganglioside G_{M3} which comprises converting Ganglioside G_{D3} included in a raw material containing lipids into Ganglioside G_{M3} in situ at a pH ranging from 6 to 8, wherein the raw material containing lipids is cream or a milk product prepared from cream, and wherein the conversion is carried out by thermally treating the raw material.

2. The method of claim 1 wherein the thermal treatment is carried out at a temperature ranging from 60°C to 140°C.

3. The method of claim 2 wherein the thermal treatment is carried out at a temperature ranging from 80°C to 110°C.

4. The method of claim 1 wherein the raw material containing lipids contains 10 wt% or more per solids of lipids.

## Patentansprüche

1. Verfahren zur Herstellung von Gangliosid G_{M3}, welches [folgende Schritte] umfaßt:
Umwandlung von Gangliosid G_{D3}, das in einem lipidhaltigen Rohmaterial enthalten ist, zu Gangliosid G_{M3} in situ bei einem pH-Wert im Bereich vom 6 bis 8, wobei das lipidhaltige Rohmaterial Sahne oder ein aus Sahne hergestelltes Milchprodukt darstellt, und wobei die Umwandlung durch thermische Behandlung des Rohmaterials durchgeführt wird.

2. Verfahren nach Anspruch 1, wobei die thermische Behandlung bei einer Temperatur im Bereich von 60°C bis 140°C durchgeführt wird.

3. Verfahren nach Anspruch 2, wobei die thermische Behandlung bei einer Temperatur im Bereich vom 80°C bis 110°C durchgeführt wird.

4. Verfahren nach Anspruch 1, worin das lipidhaltige Rohmaterial 10 Gew.-% oder mehr Lipide, bezogen auf den Feststoffgehalt der Lipide, enthält.

## Revendications

1. Procédé de préparation du ganglioside G_{M3}, qui consiste à convertir le ganglioside G_{D3} présent dans une matière première contenant des lipides en le ganglioside G_{M3} in situ à un pH compris entre 6 et 8, dans lequel la matière première contenant des lipides est la crème ou un produit laitier préparé à partir de crème, et dans lequel la conversion est mise en oeuvre par traitement thermique de la matière première.

2. Procédé selon la revendication 1, dans lequel le traitement thermique est mis en oeuvre à une température comprise entre 60 et 140°C.

3. Procédé selon la revendication 2, dans lequel le traitement thermique est mis en oeuvre à une température comprise entre 80 et 110°C.

4. Procédé selon la revendication 1, dans lequel la matière première contenant des lipides contient en extrait sec 10 % en poids ou plus de lipides.
